Europäisches Patentamt

(19) European Patent Office

Office européen des brevets

(11) Publication number: **0 141 647**
**B1**

(12) **EUROPEAN PATENT SPECIFICATION**

(45) Date of publication of patent specification: **06.09.89**

(51) Int. Cl.⁴: **G 01 N 33/52,** G 01 N 31/22, G 01 N 33/84

(21) Application number: **84307506.0**

(22) Date of filing: **31.10.84**

(54) Reflective particle-containing analysis composition and device.

(30) Priority: **07.11.83 US 549349**

(43) Date of publication of application:
**15.05.85 Bulletin 85/20**

(45) Publication of the grant of the patent:
**06.09.89 Bulletin 89/36**

(84) Designated Contracting States:
**BE CH DE FR GB IT LI NL SE**

(56) References cited:
EP-A-0 010 615
EP-A-0 041 175
GB-A-2 085 581
US-A-4 356 149
US-A-4 381 921

CHEMICAL ABSTRACTS, vol. 97, no. 15, 11th October 1982, page 333, abstract no. 123284f, Columbus, Ohio, US; B.P. BUBNIS et al.: "Substituent effects on complexation and extraction of alkali metals with chromogenic crown ethers" & ANAL. CHIM. ACTA 1982, 139, 307-13

(73) Proprietor: **TECHNICON INSTRUMENTS CORPORATION(a Delaware corporation)**
**511 Benedict Avenue**
**Tarrytown, New York 10591 (US)**

(72) Inventor: **Kumar, Anand**
**1 Duelk Avenue**
**Monroe New York 10950 (US)**
Inventor: **Leong, Koon-Wah**
**71 Van Cortlandt Avenue**
**Ossining New York 10562 (US)**

(74) Representative: **Wain, Christopher Paul et al**
**A.A. THORNTON & CO. Northumberland House**
**303-306 High Holborn**
**London WC1V 7LE (GB)**

(56) References cited:
**M.C. Fedarko, "The Use of C-13 Relasation Times to Investigate Molecular Motion of Macrocyclic Antibiotics Upon Polassium Ion Complex Formation", Journal of Magnetic Resonance 12, 30-35 (1973)**

**Kunststoff-Taschenbuch ISBN 3-446-13831-5 page 45-46 (1983)**

**Data sheet "Barostab V220 HC-F/DOP" (12/82)**

Courier Press, Leamington Spa, England.

## Description

This invention relates generally to the field of fluid analysis by reagent compositions and, more particularly to reagent compositions (and devices containing them) which are contacted with the fluid in which constituent(s) are to be determined and a detectable change is effected by chemical or other reaction.

Many organic liquids are not miscible with water, and when such liquids are added to water, two layers are formed. If an aqueous solution containing two dissolved solutes is shaken with an immiscible organic liquid and allowed to stand until the two solvent layers settle out, then if the organic liquid has a much greater affinity for one of the dissolved solutes than does water, most or all of that solute will pass from the aqueous phase to the organic phase. Thus, this solute is extracted. If the other solute prefers the aqueous phase to the organic, it will not be extracted. Eisenman, et al, *J. Membrane Biol.*, 1:294—345 (1969) discloses the selective extraction of cations from aqueous solutions into organic solvents by macrotetralide actin antibiotics. The experiments involve shaking an organic solvent phase containing the antibiotics with aqueous solutions containing various cationic salts of a lipid soluble colored anion. The intensity of color of the organic phase is then measured spectrophotometrically to indicate how much salt has been extracted. Phase transfer has also been studied by Dix. et al. *Angew. Chem. Int. Ed. Engl.*, *17*: 857 (1978) and in reviews including Burgermeister, et al. *Pop. Curr. Chem., 69*: 91 (1977); Yu, et al, *Membrane Active. Complexones*, Elsevier, Amsterdam (1974); and Duncan, *Calcium in Biological Systems*, Cambridge University Press (1976).

Most solid state chemistry elements are monitored by reflectance methods and include a plurality of layers combined in various laminar configurations. Usually, each of these is a discrete, separate layer which performs only a single function and is bound to the other layers. The reagent layer incorporates a composition which includes some or all of the reagents necessary for analysis of a particular body fluid constituent, therapeutic drug or other analyte in order to provide a measurable response. See, Walter, *Anal. Chem., 55*: 498A (1983) and Shirey *Clin. Biochem., 16*: 147—155 (1983). In such elements incident light passes through a very uniform translucent reagent layer which requires carefully defined dimensions (thickness) and is absorbed to an extent dictated by the analyte/reagent composition. A smooth opaque reflective layer laminated to the opposite side of the reagent layer reflects the unabsorbed light back through the reagent layer to a detector. The substantial distance between the site of the reaction and the point at which unabsorbed light is reflected creates a risk of interference by abberations in the reagent layer itself and from interfering substances in the sample under assay.

Reagent layers have been formed of hollow microspheres or beads and the necessary reagents incorporated within their interior. This approach is disclosed *inter alia*, in U.S. Patent Nos. 3,092,463; 3,926,732; 3,993,451; 4,129, 417; and 4,356,149. For example. Kitajima, et al, U. S. Patent No. 4,356,149 discloses a multilayer element in which the reagent layer is of an oil-in-water type dispersion wherein the reagent is contained in hydrophobic particles dispersed in a hydrophilie binder. The hydrophobic particles are permeable to a gas or a substance soluble in the hydrophobic solvent (of which they are formed) by diffusion or extraction. The particles are prepared from organic solvent-soluble polymers and liquid plasticizers. The organic solvent forms a solution, the reagent is dissolved or dispersed in the resulting solution, the solution or dispersion so obtained is added to an aqueous solution of a hydrophilic binder and emulsified or dispersed, and then a part or all of the organic solvent and water are removed by evaporation to form the reagent layer wherein the fine particles containing the reagent are dispersed in the hydrophilic binder. The multilayer element disclosed also includes a radiation-blocking or light reflection layer between the reagent layer and an aqueous liquid sample-spreading layer.

Analytical elements hawe recently been described for chromogenic determination of ionic analytes. Charlton, EP—A—41,175, discloses a test device for determining analytes such such as sodium or potassium. The device includes a layer of non-polar polymer, optionally with a plasticizer therefor, incorporated with an analyte-specific ionophore, suh as valinomycin. A further reagent is needed for the determination namely a detectable species such as a dye which is a counter ion for the analyte. This further reagent is either mixed with the sample or is present in a polymeric layer laminated with the ionophore-containing layer. In use, the analyte/ counter ion migrate into the non-porous solid phase towards the ionophore. A light scattering reflective backgrond can be provided as part of a separate support layer for reflectance measurements or by using reflective particles.

Also, Vogtle, et al, U.S. Patent No. 4,367,072 discloses a method for determining ionic analytes by contacting a sample with a conjugate of a crown ether and a chromogen or dyestuff. Introduction of the ionic analyte into the cavity of the crown ether causes a shift in molecular charge in the chromogen to which the crown ether is bound, thereby inducing a change in color. This conjugate can be incorporated in a test device. Nowhere does it disclose or suggest any relationship with or use of an extraction solvent.

Each of these approaches has provided an advantage of one sort or another. Solvent extraction techniques have been recognized for their advantages. The specificity of certain crown ethers for particular cations has provided potential for their chromogenic determination. Multilayer analytical elements which have been used for nonionic analytes have generally been relatively simple to use and provided adequate precision. However, they have been complex in their design and manufacture, thus requiring strict manufacturing control with associated difficulty and cost. The single attempt at a chromogenic test device for ionic analytes required that the sample be premixed with certain of the reagents, that a plasticized polymer entrap the ionophore reagent and that a plurality of superposed layers be used.

In contrast to the test compositions and elements previously described, the present invention provides compositions and devices whereby improved sensitivity can be obtained by combining a complete reagent system, a reflective component and the selective permeability feature in a single composition layer which can, for example, be read by diffuse reflectance. The absorptive and screening properties of the matrix are minimized in that light is not required to pass completely through the reagent layer in order to be reflected for detection. Simplicity in manufacture is combined with multi-functional capacity.

According to the invention, there is provided a composition for determining an ionic analyte in a liquid sample which composition comprises said reflective particles, a substance which is responsive to said analyte, and a polymeric binder; characterised in that:

(i) the composition is in the form of a single layer of material;

(ii) the solid reflective particles have substantially no void volume and are substantially non-absorbent, and are coated with (a) said substance and (b) an extraction solvent for the analyte;

(iii) said coated particles are uniformly dispersed throughout said binder which is permeable to said ionic analyte; and

(iv) said single layer of material provides a response, detectable by light reflectance, to an ionic analyte-containing sample without the necessity to have present any other reactant.

The invention further provides a test device or analytical element for determining an ionic analyte in a liquid sample, which device is a single integral matrix which comprises a single layer of a polymeric binder containing a substance which is responsive to said ionic analyte and solid reflective particles; characterised in that:

(i) said solid reflective particles have substantially no void volume and are substantially non-light absorbing at the detection wavelength, and are coated with (a) said substance and (b) an extraction solvent for the analyte;

(ii) said coated particles are uniformly dispersed throughout said binder which is permeable to said ionic analyte, and (iii) said single layer provides a response, detectable by light reflectance, to an ionic analyte-containing sample without the necessity to have present any other reactant. This device can include other layers, if desired, as support in laminar relationship with the analysis zone, but the device is completely effective in the absence of other layers to provide a quantitative detectable response to the presence of the analyte under assay. Also provided are a method of determining an analyte by contacting the device with a sample suspected of containing the analyte and observing any detectable response. Various embodiments, which include or use the composition, device and/or method are also contemplated. For example, the test material can be provided as part of a test kit. The kit comprises the packaged combination of one or more containers of or devices incorporated with the components of the test composition in any of a variety of physical formats.

In order that the invention may be more fully understood, reference is made to the accompanying drawings, wherein:

FIGURE 1 is a schematic side view of one embodiment of the device of the invention.

FIGURE 2 is a graphical illustration of the data resulting from the experiments described in Example I.

FIGURE 3 is a graphical illustration of the data resulting from the experiments described in Example II.

Description of the Preferred Embodiments

Specific terms in the following description which refer only to a particular embodiment are exemplary of all of the embodiments unless otherwise indicated.

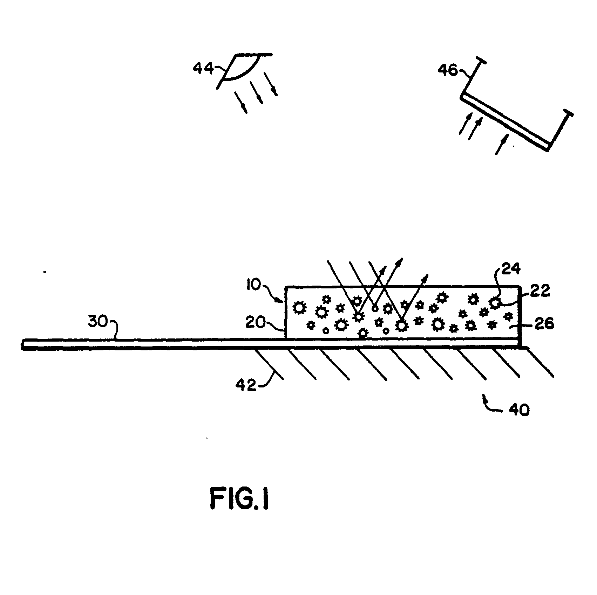

Fig. 1 illustrates the device of the present invention as it can be used with a reflectance instrument. The device 10 has an analysis layer 20. Analysis layer 20 has reflective particles 22 having reagent 24 associated with their surface in the device as illustrated. They are maintained in position by binder 26. Device 10 also has a carrier 30 which takes no part in the analysis, and is provided only for ease of handling. Liquid sample is applied to device 10 to react with reagent 24 in analysis layer 20. Device 10 is positioned in an instrument 40 on base 42. The instrument has a light source 44 which directs light on and into analysis layer 20. A detector 46 detects the amount of reflected light and the instrument reports this as a function of analyte concentration.

Sample fluids on which tests are performed include biological, physiological, industrial, environmental, and other types of liquids. Of particular interest are biological fluids such as serum, plasma, urine, cerebrospinal fluid, saliva, milk, broth and other culture media and supernatants as well as fractions of any of them. Physiological fluids of interest include infusion solutions, buffers, preservative or antimicrobial solutions and the like. Industrial liquids include fermentation media and other processing liquids used, for example, in the manufacture of pharmaceuticals, dairy products and malt beverages. Other sources of sample fluid which are tested by conventional methods are contemplated as within the meaning of this term as used and can, likewise, be assayed in accordance with the invention. Analytes of interest which are found in such sample fluids, particularly body fluids, include ionic analytes. Of particular interest are cationic analytes such as sodium, potassium and calcium.

The solid, reflective particles are optically opaque granular or particulate matter. They have no reagent or substantial internal void volume and are chemically inert to the sample, reagents and reaction for which the composition and device are intended. Within this limitation it is desirable to select particles which are not light absorbing at the detection wavelength and have as high a refractive index as possible, preferably above about 1.6. Preferably, the particles are of irregular shape and have a diameter of from about 0.1 microns to about 200 microns. Reflective, optically opaque pigments which are most preferred include basic lead hydroxide, basic lead carbonate, oxides such as titanium dioxide, zinc oxide, magnesium oxide and lead oxide and sulfates such as barium sulfate, calcium sulfate and strontium sulfate. Other materials which can be used include silica, calcite, silicates and aluminosilicates, zinc sulfide, diatomaceous earth, clay and mixtures thereof. In this invention organopolymeric particles, such as are described in U.S. Patent Nos. 4,258,001 and 4,381,921, can also be used. The combination of high refractive index and irregular particle shape enhances the scattering of incident radiation, thereby increasing analytical signal in a diffuse, reflectance colorimetric mode.

Then at least one substance that effects a detectable response in the presence of the analyte can be chosen from reagents known for their selective response to the analyte of interest. Crown ethers. so-called because of their structure, selectively react with ionic analytes, such as alkali metal ions. Spectrophotometry of alkali metals isolated by solvent extraction has been described by Takagi, et al, *Anal. Lett.*, *10*: 1115 (1977). The complexation and extraction of alkali metal ions by 4'-picrylamino-benzo-18-crown-6 derivatives has been reported by Nakamura. et al, Anal. Chem., 52: 1668—1671 (1980) and the extraction of potassium with trifluoromethyl substituted chromogenic crown ethers, including 4'-(2'',4''-dinitro-6''-trifluoromethylphenyl) aminobenzo-15-crown-5, has been reported by Pacey, et al, *Analyst, 106*: 636—640 (1981). More recently, crown ethers carrying a phenolic group, 2-hydroxyphenoxymethyl-15-crown-5 and -18-crown-6, were reported by Nakamura, et al, *Anal. Chim. Acta.*, 139: 219—227 (1982). A review of the application of crown ethers and other macrocyclic compounds in chemical analysis has been provided by Kolthoff, *Anal. Chem., 51*: 1R—22R (1979).

Extraction solvents which can be used include those known to draw analytes of interest from their usually aqueous sample environment. In a preferred embodiment they are low vapor pressure solvents such as alkyl esters of aliphatic, aromatic or phosphoric acids, alkanols, ethers (e.g., diphenyl, dioctyl, etc.) or aromatic esters.

Examples of extraction solvents useful in analysis of ionic species include phthalates such as dipentyl phthalate, mellitates such as triethyl mellitate, azelates, such as dipentyl azetate, sebacates such as di-2-ethylhexyl sebacate and phosphates such as tris-2-ethylhexyl phosphate. Higher alcohols such as octanol, decanol and dodecanol can also be used.

Binders which are suitable for use must be permeable to the analyte. The particles and analyte-responsive substance or reagent are dispersed, usually uniformly, in the binder material. The binder comprises a macromolecular substance such as proteins, gums, waxes or, preferably, polymers. The polymer can be, for example, cellulose acetate, polyvinyl chloride, nylon, starch, polycarbonate, polyvinyl alcohol, polyvinyl acetate, polyvinyl pyrrolidone, polyurethane, cellulose, cellulose nitrate or cellulose acetate butyrate. For example, the binder content of each 100 ml of the composition which is cast to form the analytical layer can include 3—9 grams (g) cellulose acetate for each 100 ml of the composition.

The invention further provides a test device or analytical element which comprises an integral matrix formed of a composition as described above. The single zone of this composition is all that is required for performance of a complete analysis. Doc. 2449—A.

This provides freedom from the requirement of multiple distinct spreading, reagent, reflection and other layers. Preferably the analysis zone is an integral homogeneous layer. The particles are coated with extraction solvent-containing analyte-responsive substance and uniformly dispersed in the binder. The integral analytical elements can be provided on a support for ease of handling, preferred supports include those of polystyrene or similar plastics. The support can be opaque, translucent or transparent to light or other energy. The devices of the present invention can be made by suitable techniques such as printing or spraying the composition onto a carrier or incorporating the solutions into film-forming liquids and allowing the combination so prepared to set or solidify, thereby imparting dimensional stability. A suitable casting solvent can include, for example, methylene chloride, ethanol and isopropanol. Such coating can be accomplished by hand, using a blade coating device, or by machine, using techniques such as dip or bead coating. The thickness of any layer and its degree of permeability are widely variable and depend on actual usage. Dry thicknesses of from about 5 microns to about 100 microns have been convenient, although more widely varying thickness may be preferable in certain circumstances.

It can be advantageous to incorporate one or more emulsifier or surfactant materials, such as anionic and non-ionic surfactant materials, in the analytical element. They can, for example, enhance solvent extraction and coatability of composition formulations and also enhance the extent and range of wetting in elements that are not easily wetted by liquid samples in the absence of an aid such as a surfactant. For example, each 100 ml of the composition which is formed into elements can include 0.4—9 grams of surface-active agents like the fatty acid partial esters of sorbital anhydrides. It can also be desirable to include materials that can render nonactive in the analysis of choice, by chemical reation or otherwise, materials potentially deleterious to such analysis.

As previously noted, many of the reagent systems which can be used provide, or can be readily adapted to provide, a detectable response, such as a color change, related to the presence or amount of the analyte under assay in the liquid sample. The element of the invention provides a change in light reflectance in the visible spectrum, thereby producing a visible color change, or a change in light reflectance outside the visible range, such as in the ultraviolet range or infrared range. The detectable response is one which can be observed tnrough tne senses directly or by use of ancillary detection means, such as a spectrophotometer or other sensing means. After the analytical result is obtained as a detectable change, it is measured, usually by passing the test element through a field in which suitable apparatus for reflectance measurement is provided. Such apparatus serves to direct a beam of energy, such as light, which is then reflected from the element back to a detector. Generally, electromagnetic radiation in the range of from about 200 to about 900 nm has been found useful for such measurements.

The following working examples describe experiments which were performed in developing the present invention. Standard commercially available reagent grade chemicals were used whenever possible.

Example I

Potassium is an important cation whose concentration in the human body is related to the muscular, respiratory, and myocardial functions as well as characteristic electrocardiographic changes. Normal serum potassium levels range from 3. 5 to 5. 3 millimols/liter (mmol/1). Levels of 10 mmol/l can be fatal. This example reports experiments in which potassium levels were quantitatively determined using an analytical element in accordance with the invention.

A 1.0 gram (g) portion of cellulose acetate (39.8% acetylation) was added to a mixture of 0.5 g Span 20 emulsifier (ICI United States, Inc., Wilmington, DE), 14 milliliters (m1) methylene chloride, 4 ml absolute ethanol, 3 ml isopropanol, 1.0 ml deionized distilled water and 60 milligrams of 4'-(2'',4''-dinitro-6''-trifluoromethylphenyl) aminobenzo-18-crown-6. The cellulose acetate was allowed to dissolve therein for at least 2 hours. Then, 1.0 g of dioctyl phthalate (extraction solvent) and 20 g titanium dioxide (2.0 um particle size) were sequentially added and the whole mixture was shaken vigorously for several minutes before it was used to form analytical elements. Another preparation was made in the same way with the exception that the dioctyl phthalate was omitted from the formulation. This provided a comparisoh with the element in accordance with the invention.

A 5 milliliter (ml) volume of the above-prepared material was placed on the uncoated side of a Gel Bond transparent polyester film (Marine Colloids, Rockland, ME) and spread to a depth of 0.5 millimeters (mm) using a conventional doctor blade. A reagent zone of about 158 square centimeters and 150 microns in thickness was produced. The film was air dried for approximately 15 minutes and the coated polyester film was then cut into 1.27 centimeter diameter circular discs.

Potassium chloride was added to aliquots of a stock solution containing 0.5 molar (M) diethanolamine hydrochloric acid (pH 9.0) to give test solutions having potassium concentrations of 2, 4, 6, 8 and 10 millimolar (mM) respectively. A solution was also made which contained no potassium, for use as a control.

Analysis of each test solution was performed by pipetting 10 microliter (u1) of the particular test solution under assay onto a separate one of the reagent-containing film discs prepared above. Each of the discs was maintained at room temperature for about 5 minutes and thereafter, the amount of potassium in each aliquot was quantified from the percent reflectance (%R) at 460 nanometers (nm) on a Macbeth series 1500 reflectance spectrometer (Macbeth, A division of Kollmorgen Corp., Color Communications, Newburgh, NY).

This procedure provided data in the form of percent reflectance units (R) which were mathematically converted by the Kubelka-Monk equation, $(1—R)^2/2R = K/S$, wherein K is the absorption coefficient and S is the scattering coefficient of the particular reflecting medium. Kartum, *Reflectance Spectroscopy*, pgs 106—111, Springer-Verlag, NY (1969). K/S is a function of the colored species concentrations and increases with the analyte concentration.

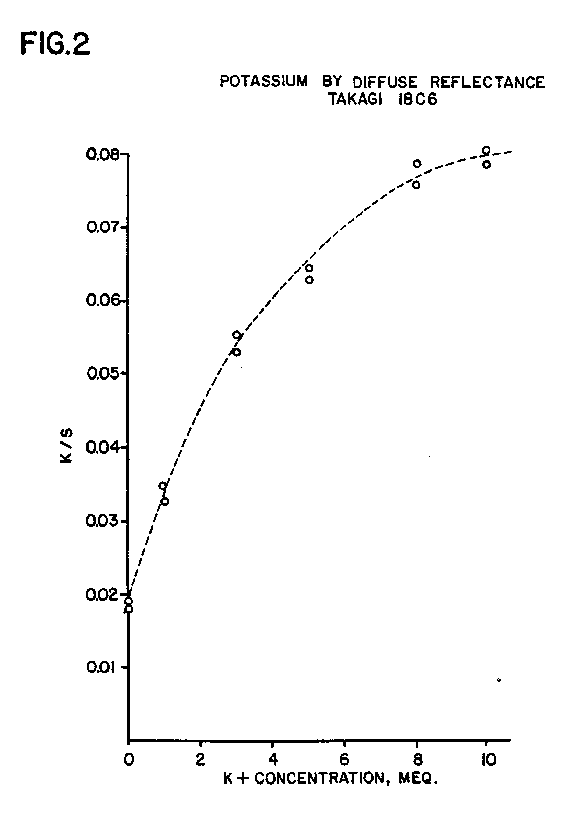

The plot of K/S versus potassium concentration ($K^+$) is shown in Fig. 2. The data reported shows the integral analytical element to provide a quantitative detectable response to the potassium concentration of each of the aliquots tested. When the dioctyl phthalate was omitted in the coating formulation, no change in percent reflectance was observed on the reagent coated discs when a test solution containing potassium was applied. This shows that a solvent medium is required to facilitate the extraction of potassium by the chromogenic crown reagent from the aqueous test solution.

Example II

Sodium is the major cation of extracellular fluid. It is an important diagnostic indicator for renal, adrenal, and other metabolic dysfunctions and has been associated with hypertension. The range of normal values for serum sodium is from 135 to 148 mmol/1. This example reports experiments in which sodium levels were quantitatively determined using an analytical element in accordance with the invention.

The composition and element used in these experiments were prepared and tested exactly as described ih Example 1 with the exception that the 4'-(2'',4''-dinitro-6''-trifluorophenylmethyl)-aminobenzo-18-crown-6 was deleted and replaced by 25 mg of 4'-(2'',4''-dihitro-6''-trifluorophenylmethyl) aminobenzo-15-crown-5.

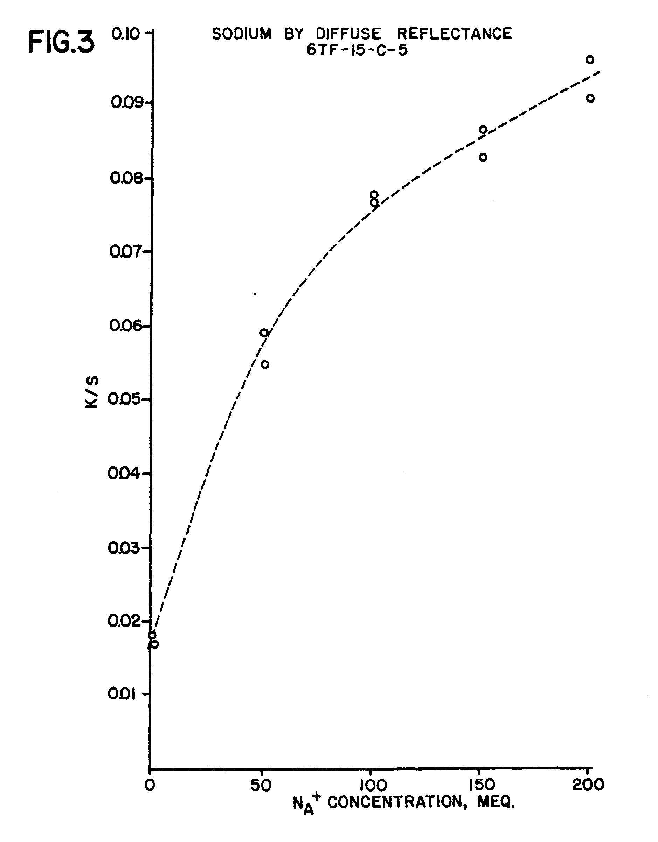

The data were obtained as reflectance units which were mathematically converted by the Kubelka-Monk equation as previously described and the plot of K/S vs. sodium concentratioh is shown in Fig. 3. The plot demonstrates a quantitative detectable response to the sodium concentration of each of the aliquots tested.

## Claims

1. A composition for determining an ionic analyte in a liquid sample which composition comprises solid reflective particles, a substance which is responsive to said analyte, and a polymeric binder; characterised in that:

(i) the composition is in the form of a single layer of material;

(ii) the solid reflective particles have substantially no void volume and are substantially non-light absorbing at the detection wavelength, and are coated with (a) said substance and (b) an extraction solvent for the analyte;

(iii) said coated particles are uniformly dispersed throughout said binder which is permeable to said ionic analyte; and

(iv) said single layer of material provides a response, detectable by light reflectance, to an ionic analyte containing sample without the necessity to have present any other reactant.

2. A composition according to claim 1, wherein the particles have a diameter of from one-tenth micron to 200 microns.

3. A composition according to claim 1 or 2, wherein the particles are of basic lead hydroxide, basic lead carbonate, titanium dioxide, zinc oxide, magnesium oxide, lead oxide, barium sulfate, calcium sulfate, strontium slfate, silica, calcite, silicates and aluminosilicates, diatomaceous earth, clay or any mixture of two or more thereof.

4. A composition according to any of claims 1 to 3, wherein the extraction solvent is a low vapor pressure solvent.

5. A composition according to claim 4, wherein the low vapor pressure solvent is an alkyl ester of an aliphatic, aromatic or phosphoric acid, an alkanol, ether or aromatic ester.

6. A composition according to claim 4, wherein the low vapor pressure solvent is a phthalate, mellitate, azelate, sebacate or phosphate.

7. A composition according to claim 6, wherein said solvent is dipentyl phthalate, tris-2-ethylhexylphosphate or di-2-ethylhexylsebacate.

8. A composition according to any of claims 1 to 7, for determining an ionic analyte, wherein the analyte responsive substance is a crown ether.

9. A composition according to claim 8, for determining potassium, wherein the crown ether is 4'-picrylaminobenzo-18-crown-6.

10. A composition according to claim 8, for determining sodium, wherein the crown ether is 4'-(2'',4''-dinitro-6''-trifluoro-methylphenyl)-aminobenzo-15-crown-5.

11. A composition according to any preceding claim, wherein the selectively permeable binder comprises at least one polymer.

12. A coomposition according to claim 4, wherein the polymer is cellulose acetate, polyvinyl chloride, nylon, polycarbonate, polyvinyl alcohol, polyvinyl acetate, polyurethane, ethyl cellulose, cellulose nitrate or cellulose acetate butyrate.

13. A method for determining an ionic analyte in a liquid sample which method comprises contacting said sample with the composition of any of claims 1 to 4, 6 or 8 to 10 and observing any detectable response detectable by light reflectance.

14. A test device for determining an ionic analyte in a liquid sample, which device is a single integral matrix which comprises a composition as claimed in claim 1.

15. A device according to claim 14, wherein the composition has the features of any of claims 2 to 12.

16. A test device according to claim 14 or 15, which further comprises a support in laminar relationship with the analysis zone.

## Patentansprüche

1. Zusammensetzung zum Bestimmen einer ionischen Analysensubstanz in einer flüssigen Probe, welche Zusammensetzung feste reflektierende Teilchen, eine auf die Analysensubstanz ansprechende Substanz und ein polymeres Bindemittel enthält, dadurch gekennzeichnet, daß

(i) die Zusammensetzung in der Form einer einzigen Materialschicht vorliegt,

(ii) die festen reflektierenden Teilchen im wesentlichen kein Leervolumen haben und bei der Erfassungswellenlänge im wesentlichen nicht lichtabsorbierend sind und überzogen sind mit (a) dieser Substanz und (b) einem Extraktionslösungsmittel für die Analysensubstanz,

(iii) die überzogenen Teilchen gleichförmig durchgehend in dem Bindemittel dispergiert sind, das für die ionische Analysensubstanz permeabel ist, und

(iv) die einzige Materialschicht eine durch Lichtreflexion erfaßbare Antwort auf eine ionische Analysensubstanz enthaltende Probe ohne die Notwendigkeit der Gegenwart irgendeines anderen Reaktionsmittels vorsieht.

2. Zusammensetzung nach Anspruch 1, bei der die Teilchen einen Durchmesser beginnend mit ein zehntel Mikrometer bis 200 Mikrometer haben.

3. Zusammensetzung nach Anspruch 1 oder 2, bei der die Teilchen aus basischem Bleihydroxid, basischem Bleicarbonat, Titandioxid, Zinkoxid, Magnesiumoxid, Bleioxid, Bariumsulfat, Calciumsulfat, Strontiumsulfat, Kieselerde, Calcit, Silicaten und Aluminosilicaten, Diatomeenerde, Ton oder irgendeinem Gemisch aus zwei oder mehreren dieser Stoffe bestehen.

4. Zusammensetzung nach einem der Ansprüche 1 bis 3, bei der das Extraktionslösungsmittel ein Lösungsmittel niedrigen Dampfdrucks ist.

5. Zusammensetzung nach Anspruch 4, bei der das Lösungsmittel niedrigen Dampfdrucks ein Alkylester einer aliphatischen, aromatischen oder Phosphorsäure, ein Alkanol, Ether oder aromatischer Ester ist.

6. Zusammensetzung nach Anspruch 4, bei der das Lösungsmittel niedrigen Dampfdrucks ein Phthalat, Mellitat, Azelat, Sebacat oder Phosphat ist.

7. Zusammensetzung nach Anspruch 6, bei der das Lösungsmittel Dipentylphthalat, Tris-2-ethylhexylphosphat oder Di-2-ethylhexylsebacat ist.

8. Zusammensetzung nach einem der Ansprüche 1 bis 7, zum Bestimmen einer ionischen Analysensubstanz, bei der die auf die Analysensubstanz ansprechende Substanz ein Kronenether ist.

9. Zusammensetzung nach Anspruch 8, zum Bestimmen von Kalium, bei dem der Kronenether eine 4'-Picrylaminobenzo-18-krone-6 ist.

10. Zusammensetzung nach Anspruch 8, zum Bestimmen von Natrium, bei dem der Kronenether eine 4'-(2'',4''-Dinitro-6''-trifluormethylphenyl)aminobenzo-15-krone-5 ist.

11. Zusammensetzung nach einem vorstehenden Anspruch, bei der das selektiv, permeable Bindemittel wenigstens ein Polymeres enthält.

12. Zusammensetzung nach Anspruch 4, bei der das Polymere Celluloseacetat, Polyvinylchlorid, Nylon, Polycarbonat, Polyvinylalkohol, Polyvinylacetat, Polyurethan, Ethylcellulose, Cellulosenitrat oder Celluloseacetatbutyrat ist.

13. Verfahren zum Bestimmen einer ionischen Analysensubstanz in einer flüssigen Probe, welches Verfahren die Kontaktierung der Probe mit der Zusammensetzung nach einem der Ansprüche 1 bis 4, 6 oder 8 bis 10 und Beobachtung irgend einer durch Lichtreflexion erfaßbaren Antwort umfaßt.

14. Prüfvorrichtung zum Bestimmen einer ionischen Analysensubstanz in einer flüssigen Probe, welche Vorrichtung eine einzige integrale Matrix ist, die eine Zusammensetzung wie beansprucht im Anspruch 1 enthält.

15. Vorrichtung nach Anspruch 14, bei der die Zusammensetzung die Merkmale von irgend einem der Ansprüche 2 bis 12 aufweist.

16. Prüfvorrichtung nach Anspruch 14 oder 15, die ferner eine Stütze in laminarer Beziehung mit der Analysenzone enthält.

## Revendications

1. Composition pour doser un analyte ionique dans un échantillon liquide, laquelle composition comprend des particules réfléchissantes pleines. une substance sensible audit analyte et un liant polymère; caractérisée en ce que:

   (i) la composition se présente sous la forme d'une couche unique de matériau;

   (ii) les particules réfléchissantes pleines n'ont pratiquement aucun volume creux et n'absorbent pratiquement pas la lumière à la longueur d'onde de détection, et sont revêtues (a) de ladite substance, et (b) d'un solvant d'extraction de l'analyte;

   (iii) lesdites particules revêtues sont uniformément dispersées dans tout le volume dudit liant, lequel est perméable audit analyte ionique; et

   (iv) ladite couche unique de matériau donne une réponse, décelable par l'intermédiaire du facteur de réflexion lumineuse, à un échantillon contenant l'analyte ionique, sans que la présence d'un quelconque autre réactif soit nécessaire.

2. Composition selon la revendication 1, dans laquelle les particules ont un diamètre de 0,1 à 200 µm.

3. Composition selon la revendication 1 ou 2, dans laquelle les particules sont constituées d'hydroxyde basique de plomb, de carbonate basique de plomb, de dioxyde de titane, d'oxyde de zinc, d'oxyde de magnésium, d'oxyde de plomb, de sulfate de baryum, de sulfate de calcium, de sulfate de strontium, de silice, de calcite, de silicates et d'aluminosilicates, de terre de diatomées, d'argile ou d'un quelconque mélange d'au moins deux de ces composés.

4. Composition selon l'une quelconque des revendications 1 à 3, dans laquelle le solvant d'extraction est un solvant ayant une faible pression de vapeur.

5. Composition selon la revendication 4, dans laquelle le solvant à faible pression de vapeur est un ester alkylique d'un acide aliphatique, aromatique ou phosphorique, un alcanol, un éther ou un ester aromatique.

6. Composition selon la revendication 4. dans laquelle le solvant à faible pression de vapeur est un phtalate, un mellitate, un azélate, un sébaçate ou un phosphate.

7. Composition selon la revendication 6, dans laquelle ledit solvant est le phtalate de dipentyle, le phosphate de tris-2-éthylhexyle ou le sébaçate de di-2-éthylhexyle.

8. Composition selon l'une quelconque des revendications 1 à 7, pour doser un analyte ionique, dans laquelle la substance sensible à l'analyte est un éther-couronne.

9. Composition selon la revendication 8, pour doser le potassium, dans laquelle l'éther-couronne est le 4'-picrylaminobenzo-18-couronne-6.

10. Composition selon la revendication 8, pour doser le sodium, dans laquelle l'éther couronne est le 4'(2'',4''-dinitro-6''-trifluorophénylméthyl)aminobenzo-15-couronne-5.

11. Composition selon l'une quelconque des revendications précédentes, dans laquelle le liant sélectivement perméable comprend au moins un polymère.

12. Composition selon la revendication 4, dans laquelle le polymère est l'acétate de cellulose, le poly(chlorure de vinyle), le nylon, le polycarbonate, le poly(alcool vinylique), le poly(acétate de vinyle), le polyurêthanne, l'éthylcellulose, le nitrate de cellulose ou l'acétobutyrate de cellulose.

13. Procédé pour doser un analyte ionique dans un échantillon liquide, lequel procédé consiste à mettre en contact ledit échantillon avec la composition selon l'une quelconque des revendications 1 à 4, 6 ou 8 à 10, et à observer toute réponse décelable, décelable par l'intermédiaire du facteur de réflexion lumineuse.

14. Dispositif d'essai pour doser un analyte ionique dans un échantillon liquide, lequel dispositif est une matrice intégrale unique comprenant une composition selon la revendication 1.

15. Dispositif selon la revendication 14, dans lequel la composition présente les caractéristiques de l'une quelconque des revendications 2 à 12.

16. Dispositif d'essai selon la revendication 14 ou 15, qui comprend en outre un support en relation de stratification avec la zone d'analyse.

FIG.1

# FIG.2

POTASSIUM BY DIFFUSE REFLECTANCE
TAKAGI 18C6

FIG.3

SODIUM BY DIFFUSE REFLECTANCE
6TF-15-C-5